# EUROPEAN PATENT APPLICATION

(11) **EP 1 783 099 A1**
(43) Date of publication of application: **09.05.2007**
(21) Application number: 05735153.8
(22) Date of filing: 05.04.2005
(51) Int. Cl.: C01B 33/46, C10G 11/02, B01J 21/06, B01J 21/12

(54) **MICROPOROUS AMORPHOUS MATERIAL, PREPARATION METHOD THEREOF AND USE OF SAME IN THE CATALYTIC CONVERSION OF ORGANIC COMPOUNDS**

(30) Priority: 07.04.2004 ES 200400968
(71) Applicant: Consejo Superior de Investigaciones Cientificas, E-28006 Madrid (ES); UNIVERSIDAD POLITECNICA DE VALENCIA, 46022 Valencia (ES)
(72) Inventor: Corma Canos, A., Instituto de Tecnología Química, Avda. Los Naranjos,s/n, E-46022 Valencia (ES); Diaz Cabanas, M.J., Inst. de Tecnología Quimica, Avda. Los Naranjos,s/n, E-46022 Valencia (ES)
(74) Representative: Ungria Lopez, Javier
(86) International application number: PCT/ES2005/070039
(87) International publication number: WO 2005/097679

(57) **Abstract**

The invention relates to a microporous amorphous material which is **characterised in that** it has a chemical composition in the calcined and anhydrous state which can be represented by the empirical formula x (M_{1/n}XO₂) : y YO₂ : SiO₂, wherein: x has a value of less than 0.2 and can be equal to zero; y has a value of less than 0.2 and can be equal to zero; M is selected from among H⁺, one or more inorganic cations having a +n charge and a mixture of same; X is one or more chemical elements in oxidation state +3; and Y is one or more elements in oxidation state +4. The inventive material is also **characterised in that** it has a uniform pore distribution, a micropore volume of greater than or equal to 0.05 cm³. g⁻¹ and a specific surface area of greater than 100 m². g⁻¹. The invention also relates to the preparation method thereof and use of same.

## Description

### Field of the Art

### Microporous molecular sieves.

### State of the Art

Zeolites are microporous crystalline materials formed by a crystal lattice of TO₄ tetrahedra which share all their vertices giving rise to a three-dimensional structure containing channels and/or cavities of molecular dimensions. They have variable composition, and T generally represents atoms with formal oxidation state of +3 or +4, such as for example Si, Ge, Ti, Al, B or Ga, among others.

The existence of channels and cavities of regular dimensions in the interior of the zeolites gives rise to a high specific area. The fact that the pore size is uniform and with a narrow distribution in these crystalline materials permits them to selectively adsorb molecules of a different size depending on the dimensions of the channels.

Moreover, the crystal structure of each zeolite, with a specific system of channels and cavities, gives rise to a characteristic X-ray diffraction pattern. So, the zeolites are differentiated among each other by their range of chemical composition and their X-ray diffraction pattern. Both characteristics (crystal structure and chemical composition) also determine the physico-chemical properties of each zeolite and its possible application in different industrial processes.

Amorphous micro- and mesoporous materials have been developed such as silicates, silicoaluminates and silicotitanates, among others, in an attempt to achieve molecular sieve properties, in other words very narrow pore distributions. In the case of microporous materials, when there existed a narrow distribution of pores, it was found that the materials could not be regarded as completely amorphous. In fact, although they did not possess any long range order, according to the X-ray diffractograms, they did indeed have short range order, as was highlighted by infrared (IR) spectroscopy, due to which the authors concluded that their material contained cores of a crystalline material, such as might be zeolite (Kragten, D. D.; Fedeyko, J. M.; Sawant, K. R.; Rimer, J. D.; Vlachos, D. G.; Lobo, R. F.; Tsapatsis, M. Journal of Physical Chemistry B (2003), 107(37), 10006-10016).

In the case of mesoporous materials, the synthesis of non-crystalline mesoporous molecular sieves developed by Kresge et al. using surfactants has opened up a new field of applications in adsorption, catalysis and electronics (Kresge, C. T.; Leonowicz, M. E.; Roth, W. J.; Vartuli, J. C.; Beck, J. S. Nature (1992), 359 (6397), 710-712).

It could thus be expected that the development of new non-crystalline mesoporous molecular sieves could open up new applications in the aforementioned fields when pores of less than 2 nm, and less than 1 nm even, are required.

### Description of the invention

The present invention relates to a microporous amorphous material characterised in that it has a chemical composition in the calcined and anhydrous state which can be represented by the following empirical formula:

x (M_{1/n}XO₂) : y YO₂ : SiO₂

wherein:
- x has a value of less than 0.2, preferably less than 0.1, and can be equal to zero,
- y has a value of less than 0.2, preferably less than 0.1, and can be equal to zero,
- M is selected from among H⁺, one or more inorganic cations having a charge +n and a mixture of same;
- X is one or more chemical elements in oxidation state +3; and
- Y is one or more elements in oxidation state +4,
which has a uniform pore distribution and a micropore volume of greater than or equal to 0.05 cm³. g⁻¹ and
which has a specific surface area of greater than 100 m². g⁻¹

In the microporous amorphous material, X is preferably selected from among Al, Ga, B, Fe, Cr and a mixture of same, and Y is preferably selected from among Ti, Ge, Sn, V and a mixture of same.

According to a preferred embodiment, said microporous amorphous material is characterised in that it has a chemical composition in the calcined and anhydrous state which can be represented by the following empirical formula:

x (M_{1/n}XO₂) : y YO₂: SiO₂

wherein:
- x has a value of less than 0.1, and can be equal to zero,
- y has a value of less than 0.1, and can be equal to zero,
- M is selected from among H⁺, one or more inorganic cations having a charge +n and a mixture of same;
- X is one or more chemical elements in oxidation state +3; and
- Y is one or more chemical elements with oxidation state +4.

The trivalent element or elements X are preferably selected from among Al, Ga, B, Fe, Cr and a mixture of same, Y is preferably selected from among Ti, Ge, Sn, V and a mixture of same, and among the inorganic cations which M can represent can be cited, for example, one or more alkaline, alkaline earth metals or mixtures of same.

According to a particular embodiment of the present invention, the microporous amorphous material can have a composition which corresponds to the formula:

x (M_{1/n}XO₂) : SiO₂

wherein:
- x has a value of less than 0.2, and can be equal to zero,
- M is selected from among H⁺, one or more inorganic cations having a charge +n and a mixture of same, and
- X is one or more chemical elements in oxidation state +3.

According to a particular embodiment of the present invention, the microporous amorphous material has a composition in the calcined and anhydrous state which corresponds to the formula:

y YO₂:SiO₂

wherein:
- y has a value of less than 0.2, preferably less than 0.1, and can be equal to zero; and
- Y is one or more chemical elements with oxidation state +4, preferably Ti, Ge, Sn, V and a mixture of same.

According to a particular embodiment of the present invention, the microporous amorphous material has a composition in the calcined and anhydrous state which corresponds to the formula:

x (HXO₂) : SiO₂

wherein:
- x has a value of less than 0.2, preferably less than 0.1, and can be equal to zero; and
- X is one or more chemical elements in oxidation state +3; preferably Al, Ga, B, Fe, Cr and a mixture of same.

According to a particular embodiment of the present invention, the microporous amorphous material has a composition in the calcined and anhydrous state which corresponds to the formula SiO₂.

The microporous non-crystalline material of the present invention, which we shall generically call MAS, has a pore distribution that is narrow and uniform in the micropore range.

Said microporous non-crystalline material is characterised in that its X-ray diffraction pattern does not display any diffraction peaks and its IR spectrum does not show bands developed in the range between 400 and 600 cm⁻¹, such bands being characteristic of crystalline silicates, such as for example zeolites.

The present invention also relates to a method for synthesising a microporous amorphous material defined earlier, comprising at least:
- preparing a reaction mixture comprising at least:
   - a source of SiO₂,
   - one or more organic compounds, and
   - water,
- subjecting said mixture to heating with or without stirring at a temperature between 80 and 200 °C, preferably between 100 and 200 °C, until achieving the formation of the amorphous material, and in which the reaction mixture has a composition, in terms of molar ratios of oxides, lying between the intervals:
- ROH/ SiO₂ = 0.01 - 3.0, preferably 0.1 - 1.0,
- H₂O/SiO₂ = 1 - 100, preferably 1 - 50,
where R is one or more organic compounds.
The reaction mixture can optionally contain a source of fluoride ions, in which case fluorhydric acid or ammonium fluoride, for example, can be used as the source of fluoride ions.

According to a particular embodiment, the method for synthesising a microporous amorphous material comprises:
- preparing a reaction mixture comprising at least:
   - a source of SiO₂,
   - a source of one or more tetravalent elements Y,
   - one or more organic compounds,
   - water,
- subjecting said mixture to heating with or without stirring at a temperature between 80 and 200 °C, preferably between 100 and 200 °C, until achieving the formation of the amorphous material, and in which the reaction mixture has a composition, in terms of molar ratios of oxides, lying between the intervals:

- ROH/ SiO₂ = 0.01 - 3.0, preferably 0.1 - 1.0,
- H₂O/SiO₂ = 1 - 100, preferably 1 - 50, and
- YO₂/SiO₂ = 0.001 - 0.2
where Y is one or more elements in an oxidation state +4, preferably Ti, Ge, Sn, V or a mixture of same, and R is one or more organic compounds. The addition of this or these elements, Y, can be done prior to heating the reaction mixture or at an intermediate moment during that heating. The reaction mixture can optionally contain a source of fluoride ions.

According to an additional particular embodiment, the method for synthesising a microporous amorphous material comprises:
- preparing a reaction mixture comprising at least:
   - a source of SiO_{2,}
   - a source of one or more trivalent elements X,
   - one or more organic compounds, and
   - water,
- subjecting said mixture to heating with or without stirring at a temperature between 80 and 200 °C, preferably between 100 and 200 °C, until achieving the formation of the amorphous material, and in which the reaction mixture has a composition, in terms of molar ratios of oxides, lying between the intervals:

- ROH/ SiO₂ = 0.01 - 3.0, preferably 0.1 - 1.0,
- H₂O/SiO₂ = 1 - 100, preferably 1 - 50, and
- X₂O₃/SiO₂ = 0.001 - 0.1,
where X is one or more elements in an oxidation state +3, preferably selected from among Al, Ga, B, Fe, Cr and a mixture of same, and R is one or more organic compounds. The addition of this or these trivalent elements can be done prior to heating the reaction mixture or at an intermediate moment during that heating.
The reaction mixture can optionally contain a source of fluoride ions. In this case, the proportion of fluoride ions in the mixture is preferably HF/SiO₂ = 0.05 -3.0.

According to an additional particular embodiment, the method for synthesising a microporous amorphous material comprises:
- preparing a reaction mixture comprising at least:
   - a source of SiO₂.
   - a source of one or more trivalent elements X,
   - one or more elements M of charge +n,
   - one or more organic compounds, and
   - water,
- subjecting said mixture to heating with or without stirring at a temperature between 80 and 200 °C, preferably between 100 and 200 °C, until achieving the formation of the amorphous material, and in which the reaction mixture has a composition, in terms of molar ratios of oxides, lying between the intervals:

- ROH/ SiO₂ = 0.01 - 3.0, preferably 0.1 - 1.0,
- H₂O/SiO₂ = 1 - 100,
- X₂O₃/SiO₂ = 0.001 - 0.1,
where X is one or more elements in an oxidation state +3, M is selected from among N⁺, one or more inorganic cations of charge +n and mixtures of same; and R is one or more organic compounds. The addition of this or these trivalent elements X can be done prior to heating the reaction mixture or at an intermediate moment during that heating.
The reaction mixture can optionally contain a source of fluoride ions. In this case, the proportion of fluoride ions in the mixture is preferably M_{1/n}F/SiO₂ = 0.05-3.0.

According to an additional particular embodiment, the method for synthesising a microporous amorphous material comprises:
- preparing a reaction mixture comprising at least:
   - a source of SiO₂,
   - a source of one or more trivalent elements X,
   - a source of one or more tetravalent elements Y,
   - one or more elements M of charge +n,
   - one or more organic compounds, and
   - water,
- subjecting said mixture to heating with or without stirring at a temperature between 80 and 200 °C, preferably between 100 and 200 °C, until achieving the formation of the amorphous material, and In which the reaction mixture has a composition, In terms of molar ratios of oxides, lying between the intervals:

- ROH/ SiO₂ = 0.01 - 3.0, preferably 0.1-1.0,
- H₂O/SiO₂ =1-100, preferably 1 - 50,
- X₂O₃/SiO₂ = 0.001 - 0.1, and
- YO₂/SiO₂ = 0.001 - 0.2
where X is one or more elements in an oxidation state +3, Y is one or more elements in an oxidation state +4, M is selected from among H⁺, one or more inorganic cations of charge +n and mixtures of same; and R is one or more organic compounds. The addition of this or these tri- and/or tetravalent elements X and Y can be done prior to heating the reaction mixture or at an intermediate moment during that heating.
The reaction mixture can optionally contain a source of fluoride ions. In this case, the proportion of fluoride ions in the mixture is preferably M_{1/n}F/SiO₂ = 0.05-3.0.

According to a preferred particular embodiment, the reaction mixture has a composition, in terms of molar ratios of oxides, lying between the intervals:
ROH/ SiO₂ = 0.1 - 1.0,
M_{1/n}F/SiO₂ = 0 - 1.0
X₂O₃SiO₂ = 0.001 - 0.05
YO₂/SiO₂ = 0.001 - 0.1
H₂O/SiO₂ = 1 - 50
where X is one or more trivalent elements selected from among Al, B, Ga, Fe and Cr and Y is one or more tetravalent elements selected from among Ti, Ge, Sn and V.

In the method of the present invention, among the sources of SiO₂ can be mentioned, for example, tetraethylorthosilicate, colloidal silica and amorphous silica.

Among the elements in oxidation state +3, Al can be used; for example, aluminium alcoxides, aluminium oxides or aluminium salts can all be used as sources of aluminium.

In any of the embodiments of the preparation method for the amorphous material described above, the organic compound R is preferably in the form of hydroxide. Alternatively it is possible to use the organic cation in salt form (for example, a halide, preferably chloride, bromide or iodide).

According to a preferred embodiment of the synthesis method, the organic compound comprises one or more amine groups. Said organic compound can also comprise one or more ammonium groups.

According to a preferred embodiment of the synthesis method, the organic compound is selected from among N(16)-methylsparteinium, 1,4-biscyclohexylpyrrolidiniumbutane hydroxide, 1,8-bisquinuclidiniumoctane hydroxide, 1,4- biscyclohexylpyrrolidiniumbutane hydroxide, hexamethonium hydroxide and tetraethylammonium hydroxide.

The heat treatment of the reaction mixture can be carried out statically or with stirring of the mixture. Once the preparation of the microporous amorphous material is finished, the solid product is separated and dried. The later calcination at temperatures between 400 and 700 °C, preferably between 450 and 600 °C, shows that the materials are thermally stable, and the decomposition of the occluded organic remains and their exit is produced, leaving the pores free.

In general, the reaction mixture has a composition which accords with the empirical formula:

a ROH : b M_{1/n}F : x X₂O₃ : y YO₂ : SiO₂ : w H₂O

where X is one or more elements in an oxidation state +3, Y is one or more elements in an oxidation state +4, M is selected from among H⁺, one or more inorganic cations of charge +n and mixtures of same; and R is one or more organic compounds, and the values a, b, x, y and w are in the ranges:
a = ROH/ SiO₂ = 0.01 - 3.0, preferably 0.1 - 1.0
b = M_{1/n}F/SiO₂ = 0 - 3.0, preferably 0.1 - 1.0
x = X₂O₃/SiO₂ = 0 - 0.01, preferably 0 - 0.05
y = YO₂/SiO₂ = 0 - 0.2, preferably 0 - 0.1
w = H₂O/SiO₂ = 1 - 100, preferably 1 - 50, more preferably 1 - 20.

The present invention also refers to a method of use of the microporous amorphous material defined earlier as a catalyst in a conversion process for organic compounds consisting of placing a feed in contact with a quantity of that catalyst.

Said process can be a process of catalytic cracking of organic compounds, preferably hydrocarbons.

According to preferred embodiments the process is selected from among a hydrocracking process, gentle hydrocracking of hydrocarbons, gentle hydrocracking of functionalised hydrocarbons, gentle hydrocracking of hydrocarbons and functionalised hydrocarbons, hydroisomerisation of olefins, an isomerisation process of light paraffins, deparaffining, isodeparaffining and an alkylation process.

In the case of an alkylation process, said alkylation can be selected from among alkylation of isoparaffins with olefins, alkylation of olefins with isoparaffins, alkylation of aromatics with olefins or alcohols, alkylation of substituted aromatics with olefins or alcohols, alkylation of thiophenic compounds with olefins or alcohols, alkylation of alkylthiophenic compounds with olefins or alcohols and alkylation of alkylbenzothiophenic compounds with olefins or alcohols. In an especially preferred manner, said alkylation is the alkylation of benzene with propylene.

According to alternative embodiments of the method of use of the microporous amorphous material, it can act as a catalyst in a process which is an acylation reaction of substituted aromatic compounds using acids, acid chlorides or organic acid anhydrides as acylating agents.

According to an additional alternative embodiment, the process is a selective oxidation of organic compounds using an oxidising agent selected from among H₂O₂, organic peroxides and organic hydroperoxides.

According to an additional alternative embodiment, the process is selected from among a Meerwein-Pondorf-Verley type oxidation reaction and a Baeyer-Villiger type oxidation reaction.

In the case of containing Ti, said microporous amorphous material can be used as a catalyst in a process of epoxidation of olefins, oxidation of alkanes, oxidation of alcohols and oxidation of organic compounds containing sulphur and which can produce sulphoxides and sulphones, using organic or inorganic hydroperoxides, such as for example, H₂O₂, tertbutylhydroperioxide, cumene hydroperoxide or molecular oxygen as oxidising agents and in ammoximation of ketones, and more specifically of cyclohexanone to cyclohexanone oxime with NH₃ and H₂O₂, or NH₃ and O₂,

In the case of containing Si, the microporous amorphous material of the present Invention can be used as a catalyst In a Baeyer-Villlger type oxidation using H₂O₂ as the oxidising agent.

### BRIEF DESCRIPTION OF THE FIGURES

Figure 1 shows the X-ray diffraction diagram of a sample of the microporous amorphous material of the invention, prepared as per example 1, using N(16)-methylspartenium, and prior to calcining.
Figure 2 shows the IR spectrum of a sample of the microporous amorphous material of the invention, known as MAS-1, prepared as per example 1, using N(16)-methylspartenium, and prior to calcining.
Figure 3 shows the pore distribution of a sample of the microporous amorphous material of the invention, prepared as per example 1, using N(16)-methylspartenium, and calcined.
Figure 4 shows the X-ray diffraction diagram of a sample of the microporous amorphous material of the invention, prepared as per example 2, using N(16)-methylspartenium, and prior to calcining.
Figure 5 shows the X-ray diffraction diagram of a sample of the microporous amorphous material of the invention containing Ti, prepared as per example 3, using N(16)-methylspartenium, and without calcining.
Figure 6 shows the X-ray diffraction diagram of a sample of the microporous amorphous material of the invention, pure silica MAS-1, and prepared as per example 4, using N(16)-methylspartenium, and without calcining.
Figure 7 shows the X-ray diffraction diagram of a sample of the microporous amorphous material of the invention, prepared as per example 5, known as MAS-2, using 1,8-bisquinuclidiniumoctane hydroxide, and without calcining.
Figure 8 shows the IR spectrum of a sample of the microporous amorphous material of the invention, prepared as per example 5, using 1,8-bisquinuclidiniumoctane hydroxide, and without calcining.

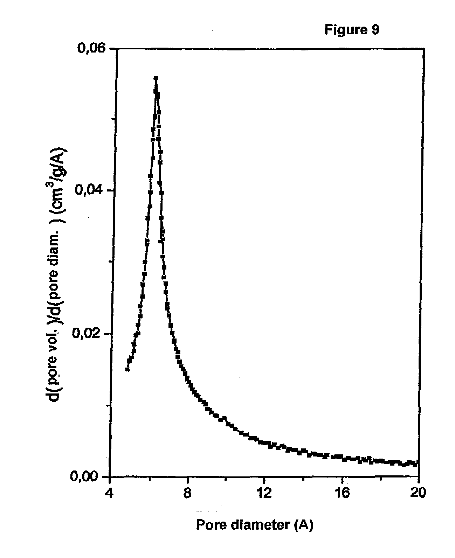

Figure 9 shows the pore distribution of a sample of the microporous amorphous material of the invention, prepared as per example 5, using 1,8-bisquinuclidiniumoctane hydroxide, and calcined.
Figure 10 shows the X-ray diffraction diagram of a sample of the microporous amorphous material of the invention, prepared as per example 6, using 1,8-bisquinuclidiniumoctane hydroxide, and without calcining.
Figure 11 shows the X-ray diffraction diagram of a sample of the microporous amorphous material of the invention, prepared as per example 7, known as MAS-3, using 1,4-biscyclohexylpyrrolidiniumbutane hydroxide, and without calcining.
Figure 12 shows the IR spectrum of a sample of the microporous amorphous material of the invention, prepared as per example 7, using 1,4-biscyclohexylpyrrolidiniumbutane hydroxide, and without calcining.
Figure 13 shows the pore distribution of a sample of the microporous amorphous material of the invention, prepared as per example 7, using 1,4-biscyclohexylpyrrolidiniumbutane hydroxide, and calcined.
Figure 14 shows the X-ray diffraction diagram of a sample of the microporous amorphous material of the invention, prepared as per example 8, using 1,4-biscyclohexylpyrrolidiniumbutane hydroxide, and without calcining.
Figure 15 shows the X-ray diffraction diagram of a sample of the microporous amorphous material of the invention, prepared as per example 9, known as MAS-4, using hexamethonium hydroxide, and without calcining.
Figure 16 shows the IR spectrum of a sample of the microporous amorphous material of the invention, prepared as per example 9, using hexamethonium hydroxide, and without calcining.
Figure 17 shows the pore distribution of a sample of the microporous amorphous material of the invention, prepared as per example 9, using hexamethonium hydroxide, and calcined.
Figure 18 shows the X-ray diffraction diagram of a sample of the microporous amorphous material of the invention, prepared as per example 10, known as MAS-5, using tetraethylammonium hydroxide, and without calcining.
Figure 19 shows the IR spectrum of a sample of the microporous amorphous material of the invention, prepared as per example 10, using tetraethylammonium hydroxide, and without calcining.
Figure 20 shows the X-ray diffraction diagram of a sample of the microporous amorphous material of the invention, prepared as per example 11, using tetraethylammonium hydroxide, and without calcining.

### EXAMPLES

### Example 1

0.272 grams of aluminium isopropoxide and 4.167 grams of tetraethylorthosilicate are hydrolysed in 11.00 grams of a solution of N(16)-methylsparteinium hydroxide with a concentration of 0.91 mols/kg. The solution obtained is kept being stirred allowing all the alcohol formed in the hydrolysis and the surplus water to evaporate. Afterwards, 0.416 g of a solution of fluorhydric acid (48.1 % of HF by weight) is then added and evaporation is continued until the reaction mixture reaches a final composition:

SiO₂ : 0.033 Al₂O₃ : 0.50 ROH : 0.50 HF : 2 H₂O

where ROH is N(16)-methylsparteinium hydroxide.

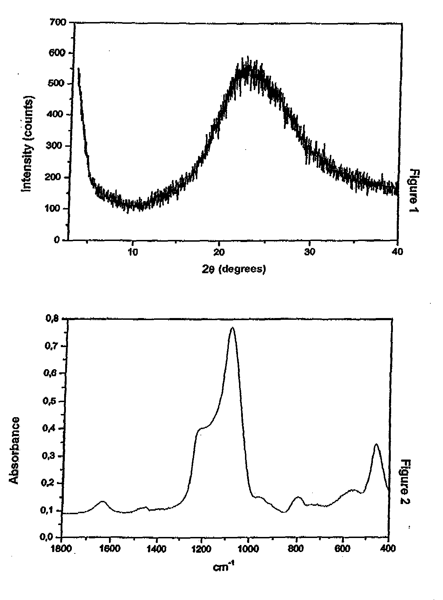

The gel is heated at 175 °C statically for 16 hours in steel autoclaves with an internal Teflon lining. The solid obtained after filtering, washing with distilled water and drying at 100 °C is MAS1. The X-ray diffraction pattern of the solid obtained, measured by the powder method using a fixed divergence grating and employing K_{α} from Cu, is shown in figure 1 and the IR spectrum in figure 2.

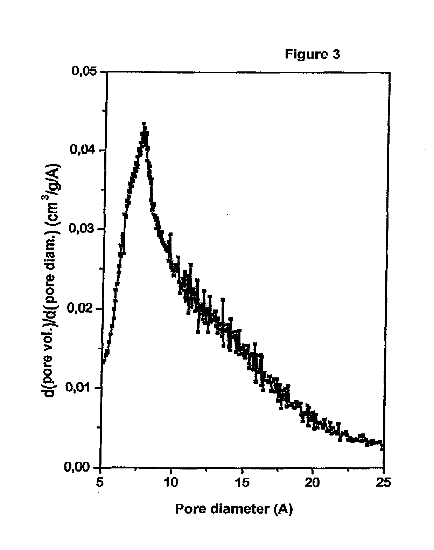

The material is calcined at 580 °C for 3 hours in an air stream in order to eliminate organic matter and the fluoride ions occluded in its interior. The solid known as MAS-1 displays a specific surface area of 738 m²/g and a micropore volume of 0.28 cm³/g. The pore diameter is 7.5 A and the pore distribution measured by Ar adsorption following the Hovartz-Kavazoe formalism is shown in figure 3.

### Example 2

52.08 g of tetraethylorthosilicate and 2.04 g of aluminium isopropoxide are hydrolysed in 132.98 g of a solution of N(16)-methylsparteinium hydroxide with a concentration of 0.94 mols/kg. The solution obtained is kept being stirred allowing the alcohol and the isopropanol formed in the hydrolysis to evaporate. Afterwards, 5.20 g of a solution of fluorhydric acid (48.1 % of HF by weight) is then added and evaporation is continued until the mixture reaches the composition:

SiO₂ : 0.02 Al₂O₃ : 0.50 ROH : 0.50 HF : 2 H₂O

where ROH is N(16)-methylsparteinium hydroxide.

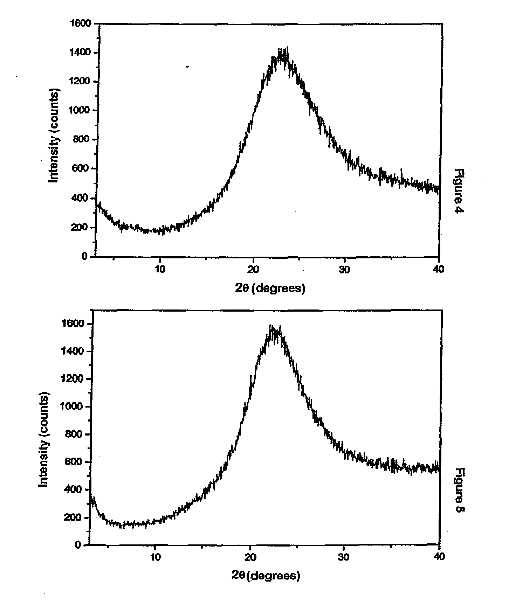

The gel is heated for 16 hours in steel autoclaves with an internal Teflon lining at 175 °C statically. The solid obtained after filtering, washing with distilled water and drying at 100 °C is MAS-1. The X-ray diffraction pattern of the solid obtained is shown in figure 4.

After calcining at 580 °C in an air stream for 3 hours, the solid, known as MAS-1, displays a specific surface area of 643 m²/g and a micropore volume of 0.24 cm³/g.

### Example 3

0.19 g of tetraethylorthotitanate and 8.33 g of tetraethylorthosilicate are hydrolysed in 24.39 g of a solution of N(16)-methylsparteinium hydroxide with a concentration of 0.86 mols/kg. The solution obtained is kept being stirred allowing all the alcohol formed in the hydrolysis and part of the surplus water to evaporate. Afterwards, 0.80 g of a solution of fluorhydric acid (50 % of HF by weight) is then added. The final composition of the synthesis gel is:

SiO₂ : 0.02 TiO₂ : 0.50 ROH : 0.50 HF : 2 H₂O

where ROH is N(16)-methylsparteinium hydroxide.

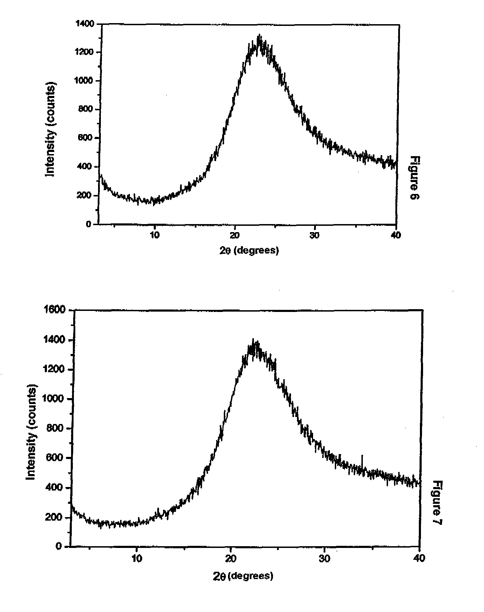

The gel is heated at 175 °C statically for 3 days in steel autoclaves with an internal Teflon lining. The solid obtained after filtering, washing with distilled water and drying at 100 °C is Ti-MAS1. The X-ray diffraction pattern of the solid obtained is shown in figure 5.

### Example 4

34.67 g of tetraethylorthosilicate are hydrolysed in 73.45 g of a solution of N(16)-methylsparteinium hydroxide with a concentration of 1.133 mols/kg, maintaining stirring and allowing all the ethanol formed in the hydrolysis to evaporate. Afterwards, 3.55 g of a solution of fluorhydric acid (46.9 % of HF by weight) is then added and evaporation is continued until the reaction mixture reaches a final composition:

SiO₂ : 0.50 ROH : 0.50 HF : 3 H₂O

where ROH is N(16)-methylsparteinium hydroxide.

After 2 days of crystallisation at 175 °C with stirring in steel autoclaves with an internal Teflon lining, pure silica MAS-1 is obtained. The X-ray diffraction pattern of the solid obtained is shown in figure 6.

### Example 5

6.34 g of tetraethylorthosilicate and 0.12 g of aluminium isopropoxide are hydrolysed in 18.80 g of a solution of 1,8-bisquinuclidiniumoctane hydroxide with a concentration of 0.81 mols of OH/kg. The solution obtained is kept being stirred allowing the alcohol and the isopropanol formed in the hydrolysis to evaporate. Afterwards, 0.61 g of a solution of fluorhydric acid (49.8 % of HF by weight) is then added and evaporation is continued until the mixture reaches the composition:

SiO₂ : 0.01 Al₂O₃ : 0.25 R(OH)₂ : 0.50 HF : 15 H₂O

where R(OH)₂ is 1,8-bisquinuclidiniumoctane hydroxide.

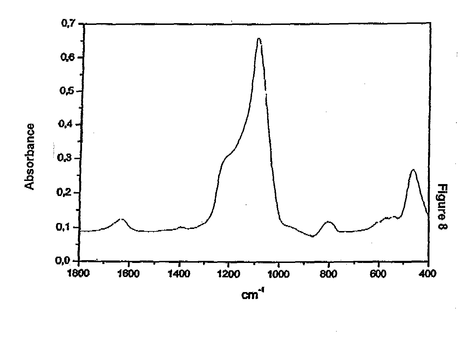

The gel is heated for 7 days in steel autoclaves with an internal Teflon lining at 175 °C with stirring. The solid obtained after filtering, washing with distilled water and drying at 100 °C is MAS-2. The X-ray diffraction pattern of the solid obtained is shown in figure 7 and the IR spectrum in figure 8.

The material is calcined at 580 °C for 3 hours in an air stream in order to eliminate organic matter and the fluoride ions occluded in its interior. The solid known as MAS-2 displays a specific surface area of 388 m²/g and a micropore volume of 0.14 cm³/g. The pore diameter is 6.0 A and the pore distribution measured by Ar adsorption following the Hovartz-Kavazoe formalism is shown in figure 3.

### Example 6

8.32 g of tetraethylorthosilicate and 0.16 g of aluminium isopropoxide are hydrolysed in 18.84 g of a solution of 1,8-bisquinuclidiniumoctane hydroxide with a concentration of 1.06 mols of OH/kg. The solution obtained is kept being stirred allowing the alcohol and the isopropanol formed in the hydrolysis to evaporate. Afterwards, 0.85 g of a solution of fluorhydric acid (46.9 % of HF by weight) is then added and evaporation is continued until the mixture reaches the composition:

SiO₂ : 0.01 Al₂O₃ : 0.50 R(OH)₂ : 0.50 HF : 15 H₂O

where R(OH)₂ is 1,8-bisquinuclidiniumoctane hydroxide.

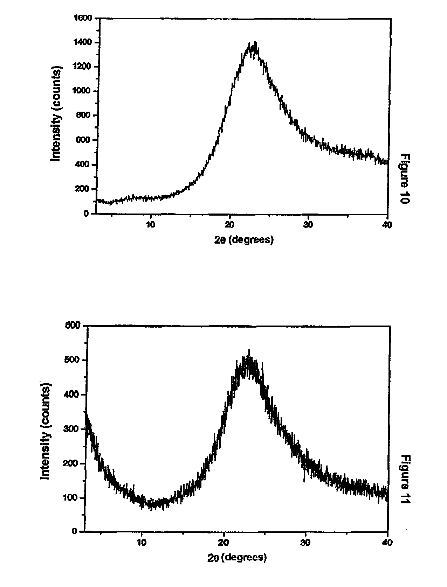

The gel is heated for 3 days in steel autoclaves with an internal Teflon lining at 150 °C with stirring. The solid obtained after filtering, washing with distilled water and drying at 100 °C is MAS-2. The X-ray diffraction pattern of the solid obtained is shown in figure 10.

### Example 7

1.27 g of aluminium isopropoxide and 12.93 g of tetraethylorthosilicate are hydrolysed in 70.04 grams of a solution of 1,4-biscyclohexylpyrrolidiniumbutane hydroxide with a concentration of 0.47 mols OH/kg. The solution obtained is kept being stirred allowing all the alcohol formed in the hydrolysis and the surplus water to evaporate. Afterwards, 1.33 g of a solution of fluorhydric acid (50 % of HF by weight) is then added and evaporation is continued until the reaction mixture reaches a final composition:

SiO₂ : 0.05 Al₂O₃ : 0.27 R(OH)₂ : 0.54 HF : 7.25 H₂O

where ROH is 1,4-biscyclohexylpyrrolidiniumbutane hydroxide.

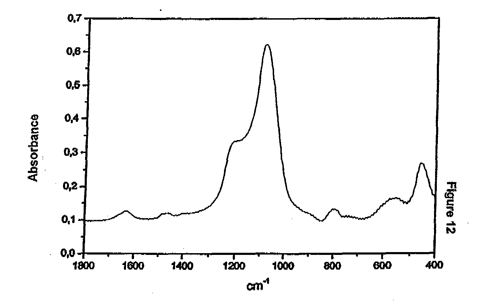

The gel is heated at 175 °C with stirring for 5 days in steel autoclaves with an internal Teflon lining. The solid obtained after filtering, washing with distilled water and drying at 100 °C is MAS-3. The X-ray diffraction pattern of the solid obtained is shown in figure 11 and the IR spectrum in figure 12.

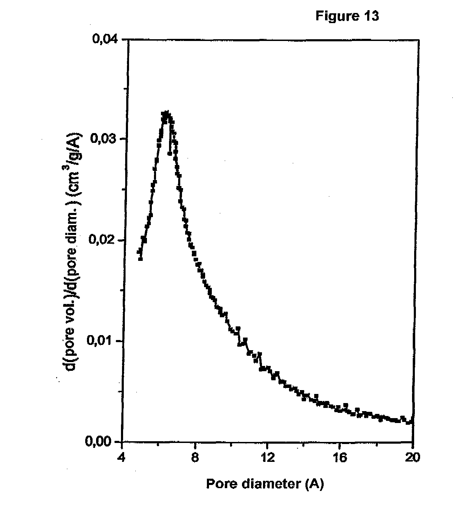

The material is calcined at 580 °C for 3 hours in an air stream in order to eliminate organic matter and the fluoride ions occluded in its interior. The solid known as MAS-1 displays a specific surface area of 418 m²/g and a micropore volume of 0.15 cm³/g. The pore diameter is 6.2 A and the pore distribution measured by Ar adsorption following the Hovartz-Kavazoe formalism is shown in figure 13.

### Example 8

8.65 g of tetraethylorthosilicate and 0.34 g of aluminium isopropoxide are hydrolysed in 38.75 g of a solution of 1,4-biscyclohexylpyrrolidiniumbutane hydroxide with a concentration of 0.28 mols OH/kg. The solution obtained is kept being stirred allowing the ethanol and the isopropanol formed in the hydrolysis to evaporate. Afterwards, 0.88 g of a solution of fluorhydric acid (50 % of HF by weight) is then added and evaporation is continued until the mixture reaches the composition:

SiO₂ : 0.02 Al₂O₃ : 0.27 R(OH)₂ : 0.54 HF : 7.25 H₂O

where ROH is 1,4-biscyclohexylpyrrolidiniumbutane hydroxide.

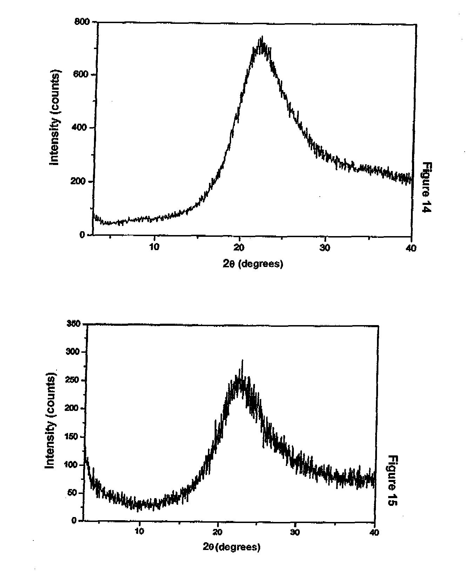

The gel is heated for 4 days in steel autoclaves with an internal Teflon lining at 175 °C with stirring. The solid obtained after filtering, washing with distilled water and drying at 100 °C is MAS-3. The X-ray diffraction pattern of the solid obtained is shown in figure 14.

### Example 9

0.24 g of GeO₂ are dissolved in 39.01 g of a solution of hexamethonium hydroxide with a concentration of 0.84 mols OH/kg. 11.84 g of tetraethylorthosilicate are hydrolysed in the solution obtained and it is kept being stirred allowing the ethanol formed to evaporate. Afterwards, 1.37 g of a solution of fluorhydric acid (48.1 % of HF by weight) is then added and evaporation is continued until the mixture reaches the composition:

0.96 SiO₂ : 0.04 GeO₂ : 0.28 R(OH)₂ : 0.56 HF : 7 H₂O

where R(OH)₂ is hexamethonium hydroxide.

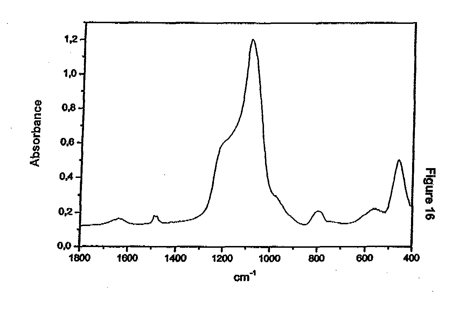

The gel is heated for 5 days in steel autoclaves with an internal Teflon lining at 135 °C with stirring. The solid obtained after filtering, washing with distilled water and drying at 100 °C is MAS-4. The X-ray diffraction pattern of the solid obtained is shown in figure 15 and the IR spectrum in figure 16.

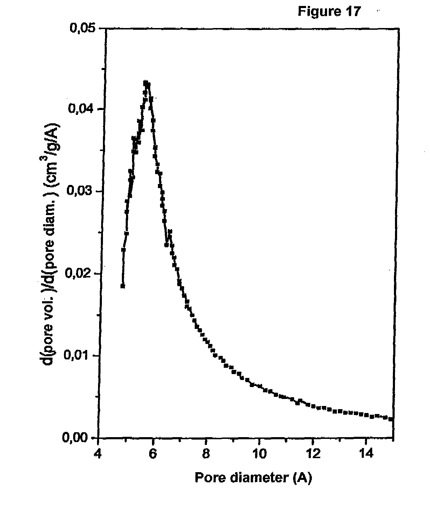

The material is calcined at 580°C for 3 hours in an air stream in order to eliminate organic matter and the fluoride ions occluded in its interior. The solid known as MAS-4 displays a specific surface area of 348 m²/g and a micropore volume of 0.13 cm³/g. The pore diameter is 5.5 A and the pore distribution measured by Ar adsorption following the Hovartz-Kavazoe formalism is shown in figure 17.

### Example 10

19.9 g of tetraethylorthosilicate are added to 14.39 g of an aqueous solution of tetraethylammonium hydroxide (TEAOH) at 40 % by weight and 3 g of water, and the mixture is stirred. A solution is then added containing 0.32 g of metallic aluminium (99.95 %) previously dissolved in 9 g of TEAOH (40 %). The mixture is left to evaporate with stirring until complete elimination of the ethanol coming from the hydrolysis of the TEOS plus the quantity of water necessary for reaching the final composition that is stated. Finally, 2.15 g of an aqueous solution of fluorhydric acid (48 % of HF by weight) is added. The composition of the gel is:

SiO₂ : 0.062 Al₂O₃ : 0.665 ROH : 0.54 HF : 7 H₂O

where ROH is tetraethylammonium hydroxide.

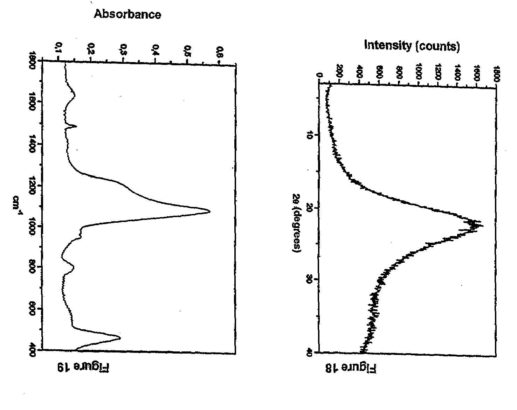

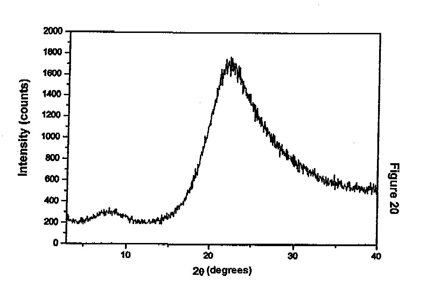

The mixture obtained is introduced into an autoclave provided with an internal polytetrafluorethylene lining and is heated at 140 °C for 6 days in a stove provided with a rotation system. After that time, it is recovered by means of filtering, washed with water and then dried at 100 °C, giving 27.9 g of solid per 100 g of gel. The solid obtained is MAS-5 and its X-ray diffraction pattern of the solid obtained is shown in figure 18 and the IR spectrum in figure 19.

The material is calcined at 580 °C for 3 hours in an air stream in order to eliminate organic matter and the fluoride ions occluded in its interior.

### Example 11

15.00 grams of tetraethylorthosilicate are hydrolysed in 16.36 grams of a solution of tetraethylammonium hydroxide (TEAOH) at 40 % by weight and 3 g of water, and the mixture is stirred. The solution obtained is kept being stirred allowing all the alcohol formed in the hydrolysis and the surplus water to evaporate. Afterwards, 1.56 g of a solution of fluorhydric acid (50 % of HF by weight) is then added and evaporation is continued until the mixture reaches a final composition:

SiO₂ : 0.54 ROH : 0.54 HF : 7 H₂O

where ROH is tetraethylammonium hydroxide.

The gel is heated at 175 °C with stirring for 4 hours in steel autoclaves with an internal Teflon lining. The solid obtained after filtering, washing with distilled water and drying at 100 °C is MAS-5 and its X-ray diffraction pattern is shown in figure 20.

### Example 12

This example shows the catalytic activity of a bifunctional catalyst formed from an acid function (MAS, prepared as per example 2) and a hydrogenating-dehydrogenating function (Pt 1.0 % by weight), introduced by impregnation starting from an aqueous solution of hexachloroplatinic acid, for the hydrocracking of n-hexadecane. The reaction was carried out in a fixed bed continuous reactor at 270 °C, pressure 40 bars, with a molar ratio of H₂/hexadecane of 95 and a contact time (W/F) of 0.27 hours. Under these conditions the conversion of n-hexadecane was 84.6 % with a selectivity to C₁₆ Isomers of 2.3 % and a selectivity to hydrocracking products of 97.7 %. No products with one or two carbon atoms were detected among the hydrocracking products. The yield of C₅-C₇ products was 35.8 %.

### Example 13

This example shows the catalytic activity of a bifunctional catalyst formed from an acid function (MAS, prepared as per example 2) and a hydrogenating function (Pt 1 % by weight), introduced by impregnation starting from an aqueous solution of hexachloroplatinic acid, for the hydrocracking of a hydrotreated gasoil containing 10.6 % by weight of hydrocarbons with a boiling point between 250 °C and 380 °C, and 89.4 % with boiling point above 380 °C, and a sulphur content of 87 ppm. The reaction conditions were: 370 °C, 0.2 hours contact time (W/F), pressure 50 bars and volume ratio under normal conditions of H_{2/gasoil} = 988.

After eight hours under these reaction conditions the conversion (380 °C⁻) was 56.4 % with yields to gases of 0.7%, to naphtha (65° - 150° C) of 12.5%, to kerosene (150° - 250° C) of 20.1% and to mean distillates (250° - 380° C) of 23.0%.

### Example 14

This example shows the catalytic activity for catalytic cracking of an MAS material prepared according to example 2, in which n-decane is used as reagent. The reaction conditions were: atmospheric pressure, weight ratio of catalyst/feed of 0.70, temperature of 500°C and reaction time of 60 seconds. Under these conditions the conversion was 33%.

### Example 15

This example shows the catalytic activity for cracking of a vacuum gasoil, of the MAS material prepared as per example 2. The reaction conditions were: atmospheric pressure, weight ratio of catalyst/feed of 0.65, reaction temperature of 500°C and reaction time of 60 seconds. The conversion was 60% by weight, with a yield to gases, gasoline, diesel and coke of 19.1, 23.2, 14.0 and 3.7 %, respectively, the propylene/propane ratio in the gases being 4.9, for a propylene yield of 7%.

## Claims

1. A microporous amorphous material **characterised in that** it has a chemical composition in the calcined and anhydrous state which can be represented by the following empirical formula:
x (M_{1/n}XO₂) : y YO₂ : SiO₂
wherein:
- x has a value of less than 0.2, and can be equal to zero,
- y has a value of less than 0.2, and can be equal to zero,
- M is selected from among H⁺, one or more inorganic cations having a charge +n and a mixture of same;
- X is one or more chemical elements in oxidation state +3; and
- Y is one or more elements in oxidation state +4,
which has a uniform pore distribution and a micropore volume of greater than or equal to 0.05 cm³.g⁻¹ and
which has a specific surface area of greater than 100 m². g⁻¹

2. A microporous amorphous material, according to claim 1, **characterised in that** it has a chemical composition in the calcined and anhydrous state which can be represented by the following empirical formula:
x (M_{1/n}XO₂) : y YO₂ : SiO₂
wherein:
- x has a value of less than 0.1, and can be equal to zero,
- y has a value of less than 0.1, and can be equal to zero,
- M is selected from among H⁺, one or more inorganic cations having a charge +n and a mixture of same;
- X is one or more chemical elements in oxidation state +3; and
- Y is one or more chemical elements with oxidation state +4.

3. A microporous amorphous material according to claim 1 or 2, **characterised in that** X is selected from among Al, Ga, B, Fe, Cr and a mixture of same.

4. A microporous amorphous material according to claim 1 or 2,
**characterised in that** Y is selected from among Ti, Ge, Sn, V and a mixture of same.

5. A microporous amorphous material according to one of claims 1, 2 or 3, **characterised in that** its composition corresponds to the formula:
x (M_{1/n}XO₂) : SiO₂
wherein:
- x has a value of less than 0.2, and can be equal to zero,
- M is selected from among H⁺, one or more inorganic cations having a charge +n and a mixture of same, and
- X is one or more chemical elements in oxidation state +3.

6. A microporous amorphous material according to one of claims 1, 2 or 3, **characterised in that** its composition in the calcined and anhydrous state can be represented by the formula:
y YO₂: SiO₂
wherein:
- y has a value of less than 0.2, and can be equal to zero; and
- Y is one or more chemical elements with oxidation state +4.

7. A microporous amorphous material according to one of claims 1, 2 or 3, **characterised in that** its chemical composition in the calcined and anhydrous state can be represented by the empirical formula:
x (HXO₂) : SiO₂
wherein:
- x has a value of less than 0.2, and can be equal to zero; and
- X is one or more chemical elements in oxidation state +3.

8. A microporous amorphous material according to one of claims 1, 2 or 3, **characterised in that** its chemical composition in the calcined and anhydrous state is represented by the formula SiO₂.

9. A method for synthesising a microporous amorphous material defined in one of claims 1 to 8, **characterised in that** it comprises:
- preparing a reaction mixture comprising at least:
- a source of SiO₂,
- one or more organic compounds, and
- water,
- subjecting said mixture to heating with or without stirring at a temperature between 80 and 200 °C, until achieving the formation of the amorphous material, and in which the reaction mixture has a composition, in terms of molar ratios of oxides, lying between the intervals:
- ROH/ SiO₂ = 0.01 - 3.0,
- H₂O/SiO₂ = 1 - 100,
where R is one or more organic compounds.

10. A method for synthesising an amorphous material according to claim 9, **characterised in that** the reaction mixture furthermore comprises a source of fluoride ions.

11. A method for synthesising an amorphous material according to one of claims 9 or 10, **characterised in that** it comprises:
- preparing a reaction mixture comprising at least:
- a source of SiO₂.
- a source of one or more tetravalent elements Y,
- one or more organic compounds,
- water,
- subjecting said mixture to heating with or without stirring at a temperature between 80 and 200 °C, until achieving the formation of the amorphous material, and in which the reaction mixture has a composition, in terms of molar ratios of oxides, lying between the intervals:
- ROH/ SiO₂ = 0.01 - 3.0,
- H₂O/SiO₂ = 1-100, and
- YO₂/SiO₂ = 0.001 - 0.2
where Y is one or more elements in an oxidation state +4, and R is one or more organic compounds.

12. A method for synthesising an amorphous material according to claim 9 or 10, **characterised in that** it comprises:
- preparing a reaction mixture comprising at least:
- a source of SiO₂,
- a source of one or more trivalent elements X,
- one or more organic compounds, and
- water,
- subjecting said mixture to heating with or without stirring at a temperature between 80 and 200 °C, until achieving the formation of the amorphous material, and in which the reaction mixture has a composition, in terms of molar ratios of oxides, lying between the intervals:
- ROH/ SiO₂ = 0.01 - 3.0,
- H₂O/SiO₂ = 1 - 100, and
- X₂O₃SiO₂ = 0.001 - 0.1,
where X is one or more elements in an oxidation state +3 and R is one or more organic compounds.

13. A method for synthesising an amorphous material according to one of claims 9 or 10, **characterised in that** it comprises:
- preparing a reaction mixture comprising at least:
- a source of SiO₂,
- a source of one or more trivalent elements X,
- one or more elements M of charge +n,
- one or more organic compounds, and
- water,
- subjecting said mixture to heating with or without stirring at a temperature between 80 and 200 °C, until achieving the formation of the amorphous material, and in which the reaction mixture has a composition, in terms of molar ratios of oxides, lying between the intervals:
- ROH/SiO₂= 0.01-3.0,
- H₂O/SiO₂ = 1 - 100, and
- X₂O₃/SiO₂ = 0.001 - 0.1,
where X is one or more elements in an oxidation state +3, M is selected from among H⁺, one or more inorganic cations of charge +n and mixtures of same; and R is one or more organic compounds.

14. A method for synthesising an amorphous material according to one of claims 9 or 10, **characterised in that** it comprises:
- preparing a reaction mixture comprising at least:
- a source of SiO₂,
- a source of one or more trivalent elements X,
- a source of one or more tetravalent elements Y,
- one or more elements M of charge +n,
- one or more organic compounds, and
- water,
- subjecting said mixture to heating with or without stirring at a temperature between 80 and 200 °C, until achieving the formation of the amorphous material, and in which the reaction mixture has a composition, in terms of molar ratios of oxides, lying between the intervals:
- ROH/ SiO₂= 0.01 - 3.0,
- H₂O/SiO₂ =1-100,
- X₂O₃/SiO₂ =0.001 - 0.1,
- YO₂/SiO₂ = 0.001 - 0.2
where X is one or more elements in an oxidation state +3, Y is one or more elements in an oxidation state +4, M is selected from among H⁺, one or more inorganic cations of charge +n and mixtures of same; and R is one or more organic compounds.

15. A method for synthesising an amorphous material according to claim 14, **characterised in that** the reaction mixture has a composition, in terms of molar ratios of oxides, lying between the intervals
ROH/SiO₂=0.1 - 1.0,
X₂O₃/SiO₂ = 0.001 - 0.05
YO₂/SiO₂ = 0.001 - 0.1
H₂O/SiO₂ =1-50
where X is one or more trivalent elements selected from among Al, B, Ga, Fe and Cr and Y is one or more tetravalent elements selected from among Ti, Ge, Sn and V.

16. A method for synthesising an amorphous material according to claim 14, **characterised in that** the reaction mixture has a composition which accords with the empirical formula:
a ROH : b M_{1/n}F : x X₂O₃ : y YO₂ : SiO₂ : w H₂O
where X is one or more elements in an oxidation state +3, Y is one or more elements in an oxidation state +4, M is selected from among H⁺, one or more inorganic cations of charge +n and mixtures of same; and R is one or more organic compounds,
and the values a, b, x, y and w are in the ranges:
a = ROH/ SiO₂ = 0.1 - 3.0,
b = M_{1/n}F/SiO₂ = 0.1 - 3.0,
x = X₂O₃/SiO₂ = 0 - 0.05,
y = YO₂/SiO₂ = 0 - 0.1, and
w = H₂O/SiO₂ =1-50,

17. A method for synthesising an amorphous material according to one of claims 9 to 16, **characterised in that** the mixture is subjected to heating with or without stirring at a temperature between 100 and 200 °C.

18. A method for synthesising an amorphous material according to one of claims 9 to 17, **characterised in that** the organic compound R is in hydroxide form.

19. A method for synthesising an amorphous material according to one of claims 9 to 17, **characterised in that** the organic compound comprises one or more amine groups.

20. A method for synthesising an amorphous material according to one of claims 9 to 17, **characterised in that** the organic compound comprises one or more ammonium groups.

21. A method for synthesising an amorphous material according to one of claims 9 to 17, **characterised in that** the organic compound is selected from among N(16)-methylsparteinium, 1,4-biscyclohexylpyrrolidiniumbutane hydroxide, 1,8-bisquinuclidiniumoctane hydroxide, 1,4-biscyclohexylpyrrolidiniumbutane hydroxide, hexamethonium hydroxide and tetraethylammonium hydroxide.

22. Method of use of an amorphous material of any of claims 1 to 14, as catalyst in a conversion process for organic compounds consisting of placing a feed in contact with a quantity of that catalyst.

23. Method of use according to claim 22, **characterised in that** the process can be a process of catalytic cracking of organic compounds.

24. Method of use of an amorphous material according to claim 23, **characterised in that** said organic compounds are hydrocarbons.

25. Method of use of an amorphous material according to claim 22, **characterised in that** the process is selected from among a hydrocracking process, gentle hydrocracking of hydrocarbons, gentle hydrocracking of functionalised hydrocarbons, gentle hydrocracking of hydrocarbons and functionalised hydrocarbons, hydroisomerisation of olefins, an isomerisation process of light paraffins, deparaffining, isodeparaffining and an alkylation process.

26. Method of use of an amorphous material according to claim 25, **characterised in that** the alkylation process is selected from among alkylation of isoparaffins with olefins, alkylation of olefins with isoparaffins, alkylation of aromatics with olefins or alcohols, alkylation of substituted aromatics with olefins or alcohols, alkylation of thiophenic compounds with olefins or alcohols, alkylation of alkylthiophenic compounds with olefins or alcohols, alkylation of alkylbenzothiophenic compounds with olefins or alcohols.

27. Method of use of an amorphous material according to claim 25, **characterised in that** the alkylation process is the alkylation of benzene with propylene.

28. Method of use of an amorphous material according to claim 22, **characterised in that** the process is an acylation reaction of substituted aromatic compounds using acids, acid chlorides or organic acid anhydrides as acylating agents.

29. Method of use of an amorphous material according to claim 22, **characterised in that** the process is a selective oxidation of organic compounds using an oxidising agent selected from among H₂O₂, organic peroxides and organic hydroperoxides.

30. Method of use of an amorphous material according to claim 22, **characterised in that** the process is selected from among a Meerwein-Pondorf-Verley type oxidation reaction and a Baeyer-Villiger type oxidation reaction.

31. Method of use of an amorphous material according to claim 22, **characterised in that** said amorphous material contains Ti and the process is selected from among:
- a process of epoxidation of olefins,
- oxidation of alkanes, oxidation of alcohols,
- oxidation of organic compounds containing sulphur and which can produce sulphoxides and sulphones, using organic or inorganic hydroperoxides or molecular oxygen as oxidising agents and
- ammoximation of ketones.

32. Method of use of an amorphous material according to claim 22, **characterised in that** said amorphous material contains Sn and the process is an oxidation in Baeyer-Villiger reactions using H₂O₂ as oxidising agent.
